(19) Europäisches Patentamt
European Patent Office
Office européen des brevets

(11) Veröffentlichungsnummer: **0 136 451**
**A2**

# (12) EUROPÄISCHE PATENTANMELDUNG

(21) Anmeldenummer: **84108846.1**

(22) Anmeldetag: **26.07.84**

(51) Int. Cl.[4]: **C 07 C 43/29**
C 07 C 43/267, C 07 C 43/174
C 07 C 43/168, C 07 D 213/64
C 07 D 317/54, A 01 N 31/04
A 01 N 31/14, A 01 N 43/30
A 01 N 43/40

(30) Priorität: **12.09.83 DE 3333239**

(43) Veröffentlichungstag der Anmeldung:
**10.04.85 Patentblatt 85/15**

(84) Benannte Vertragsstaaten:
**BE CH DE FR GB IT LI LU NL SE**

(71) Anmelder: **SCHERING AKTIENGESELLSCHAFT Berlin und Bergkamen**
**Müllerstrasse 170/178 Postfach 65 03 11**
**D-1000 Berlin 65(DE)**

(72) Erfinder: **Franke, Heinrich, Dr.**
**Fabeckstrasse 38**
**D-1000 Berlin 33(DE)**

(72) Erfinder: **Joppien, Hartmut, Dr.**
**Juttastrasse 18**
**D-1000 Berlin 37(DE)**

## (54) Arylmethylätherderivate, Schädlingsbekämpfungsmittel enthaltend diese Verbindungen sowie Verfahren zu ihrer Herstellung.

(57) Die Erfindung betrifft neue Arylmethylätherderivate der allgemeinen Formel

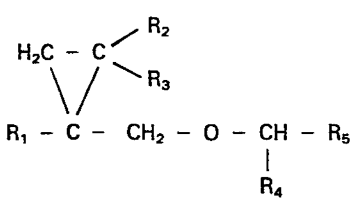

I,

in der

$R_1$ Aryl, durch $C_1$-$C_4$-Alkyl, Halogen-$C_1$-$C_4$-alkyl, Phenyl-$C_1$-$C_4$-alkyl, $C_2$-$C_4$-Alkenyl, Halogen-$C_2$-$C_4$-alkenyl, Phenyl-$C_2$-$C_4$-alkenyl, $C_2$-$C_4$-Alkinyl, Halogen-$C_2$-$C_4$-alkinyl, Phenyl-$C_2$-$C_4$-alkinyl, $C_1$-$C_4$-Alkoxy, Halogen-$C_1$-$C_4$-alkoxy, Phenyl-$C_1$-$C_4$-alkoxy, $C_2$-$C_4$-Alkenyloxy, Halogen-$C_2$-$C_4$-alkenyloxy, Phenyl-$C_2$-$C_4$-alkenyloxy, Halogen, Cyan, Nitro, Aryloxy, Halogenaryloxy, $C_1$-$C_4$-Alklyl-aryloxy oder Nitro-$C_1$-$C_4$-alkyl-aryloxy substituiertes Aryl,

$R_2$ Wasserstoff, Methyl oder Halogen,

$R_3$ Wasserstoff, Methyl oder Halogen,

$R_4$ Wasserstoff, Cyano oder Äthinyl,

$R_5$ Phenyl, Pyridyl oder durch $C_1$-$C_6$-Alkyl, Halogen-$C_1$-$C_6$-alkyl, Phenyl-$C_1$-$C_6$-alkyl, durch O-, N- oder S-Atome unterbrochenes $C_1$-$C_6$-Alkyl, $C_2$-$C_4$-Alkenyl, Halogen-$C_2$-$C_4$-alkenyl, Phenyl-$C_2$-$C_4$-alkenyl, $C_1$-$C_4$-Alkoxy, Halogen-$C_1$-$C_4$-alkoxy, Phenyl-$C_1$-$C_4$-alkoxy, $C_2$-$C_4$-Alkenyloxy, Halogen-$C_2$-$C_4$-alkenyloxy, Phenyl-$C_2$-$C_4$-alkenyloxy, Aryloxy, Halogenaryloxy, $C_1$-$C_4$-Alkyl-aryloxy, Arylamino, Halogenarylamino, $C_1$-$C_4$-Alkylarylamino, Aryl-N-$C_1$-$C_4$-alkyl-amino, Aryl-N-$C_1$-$C_4$-aryl-amino, Aroyl, Halogenaroyl, $C_1$-$C_4$-Alkylaroyl, Aryl, Halogenaryl, $C_1$-$C_4$-Alkylaryl oder Halogen ein- oder mehrfach substituiertes Phenyl oder Pyridyl darstellen, Schädlingsbekämpfungsmittel enthaltend diese Verbindungen sowie Verfahren zu ihrer Herstellung.

EP 0 136 451 A2

Die Erfindung betrifft neue Arylmethylätherderivate gemäß Oberbegriff des Anspruchs 1, ein Verfahren gemäß Oberbegriff des Anspruchs 6 zur Herstellung dieser Verbindungen sowie Schädlingsbekämpfungsmittel gemäß Oberbegriff des Anspruchs 7 enthaltend diese Wirkstoffe.

Konstitutionsanaloge Wirkstoffe gleicher Wirkungsrichtung sind bereits bekannt (DE-OS 27 09 355; DE-OS 31 17 510).

Aufgabe der vorliegenden Erfindung ist die Entwicklung neuer Wirkstoffe und Mittel mit überlegenen Eigenschaften.

Diese Aufgabe wird erfindungsgemäß durch den in den Ansprüchen gekennzeichneten Gegenstand gelöst.
Vorteilhafte Weiterbildungen der Erfindung sind in den Unteransprüchen beschrieben.

Der in der allgemeinen Formel I als $R_1$ bezeichnete Arylrest umfaßt auch die Reste 1-Naphthyl, 2-Naphthyl, Benzofuran-5-yl, Benzothiophen-5-yl, Benzofuran-6-yl, Benzothiophen-6-yl, Benzoxazol -5-yl, Benzoxazol -6-yl, Indan-5-yl, Indan-6-yl, 1,4-Benzodioxan-6-yl, 1,3-Benzodioxan-6-yl, 1,3-Benzodioxan-7-yl und 1,3-Benzodioxol-5-yl.

Die erfindungsgemäßen Verbindungen weisen überraschenderweise eine im Vergleich zu den bekannten konstitutionsanalogen Wirkstoffen überlegene insektizide und ovizide Wirkung auf und besitzen darüberhinaus den Vorteil, außerordentlich pflanzenverträglich zu sein.

Die Anwendung der erfindungsgemäßen Verbindungen kann in Konzentrationen von 0,0005 bis 5,0 %, vorzugsweise von 0,001 bis 0,1 % erfolgen, worunter das Gewicht in Gramm Wirkstoff in 100 ml Zubereitung zu verstehen ist.

Die erfindungsgemäßen Verbindungen können entweder allein,



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin

in Mischung miteinander oder mit anderen insektiziden Wirkstoffen angewendet werden. Gegebenenfalls können andere Pflanzenschutz- oder Schädlingsbekämpfungsmittel, wie zum Beispiel Insektizide, Akarizide oder Fungizide, je nach dem gewünschten Zweck zugesetzt werden.

Eine Förderung der Wirkungsintensität und der Wirkungsgeschwindigkeit kann zum Beispiel durch wirkungssteigernde Zusätze, wie organische Lösungsmittel, Netzmittel und Öle erzielt werden. Solche Zusätze lassen daher gegebenenfalls eine Verringerung der Wirkstoffdosierung zu.

Als Mischungspartner können außerdem Phospholipide verwendet werden, zum Beispiel solche aus der Gruppe Phosphatidylcholin, den hydrierten Phosphatidylcholinen, Phosphatidylethanolamin, den N-Acyl-phosphatidylethanolaminen, Phosphatidylinosit, Phosphatidylserin, Lysolecithin und Phosphatidylglycerol.

Zweckmäßig werden die gekennzeichneten Wirkstoffe oder deren Mischungen in Form von Zubereitungen, wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier- und/oder Dispergierhilfsmitteln angewandt.

Geeignete flüssige Trägerstoffe sind zum Beispiel aliphatische und aromatische Kohlenwasserstoffe, wie Benzol. Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralerden, zum Beispiel Tonsil, Silicagel, Talkum, Kaolin, Attapulgit, Kalkstein, Kieselsäure und pflanzliche Produkte, zum Beispiel Mehle.



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Haman
Sitz der Gesellschaft: Berlin und Bergkamen · Handelsregister: AG Charlottenburg 93 HRB 283 und AG Kamen HRB 0061 · Berliner Commerzbank AG, Berlin, Konto-N
108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-N
2415008, Bankleitzahl 100 700 00 · Postscheckamt Berlin West, Konto-Nr. 1175-101, Bankleitzahl 100 100 10

An oberflächenaktiven Stoffen sind zu nennen: zum Beispiel Calciumligninsulfonat, Polyäthylenalkylphenyl-äther, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil des bzw. der Wirkstoffe(s) in den verschiedenen Zubereitungen kann in weiten Grenzen variieren. Beispielsweise enthalten die Mittel etwa 10 bis 90 Gewichtsprozent Wirkstoffe, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen von etwa 100 bis 3000 Liter/ha. Eine Anwendung der Mittel im sogenannten Low-Volume und Ultra-Low-Volume-Verfahren ist ebenso möglich wie ihre Applikation in Form von sogenannten Mikrogranulaten.

Die Herstellung dieser Zubereitungen kann in an sich bekannter Art und Weise, zum Beispiel durch Mahl- oder Mischverfahren, durchgeführt werden. Gewünschtenfalls können die Einzelkomponenten auch erst kurz vor ihrer Verwendung gemischt werden, wie es zum Beispiel im sogenannten Tankmixverfahren in der Praxis durchgeführt wird.

Zur Herstellung der Zubereitungen werden zum Beispiel die folgenden Bestandteile eingesetzt:



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin.
Vorstand: Dr. Herbert Asmis. Dr. Christian Bruhn. Dr. Heinz Hannse. Horst Kramp. Dr. Klaus Pohle. Dr. Horst Witzel · Vorsitzender des Aufsichtsrats: Hans-Jürgen Hamann
Sitz der Gesellschaft Berlin und Bergkamen Handelsregister: AG Charlottenburg 93 HRB 283 und AG Kamen HRB 0061 · Berliner Commerzbank AG, Berlin, Konto-Nr 108700600. Bankleitzahl 100 400 00 Berliner Handels- und Frankfurter Bank, Berlin. Konto-Nr. 70045224. Bankleitzahl 100 202 00 Deutsche Bank Berlin AG, Konto-Nr. 2415008. Bankleitzahl 100 700 00 Postscheckamt Berlin West. Konto-Nr. 1175-101. Bankleitzahl 100 100 10

a) 80 Gewichtsprozent Wirkstoff
15 Gewichtsprozent Kaolin
5 Gewichtsprozent oberflächenaktive Stoffe auf Basis des Natriumsalzes des N-Methyl-N-oleyl-taurins und des Calciumsalzes der Ligninsulfonsäure.

b) 45 Gewichtsprozent Wirkstoff
5 Gewichtsprozent Natriumaluminiumsilikat
15 Gewichtsprozent Cetylpolyglycoläther mit 8 Mol Äthylenoxid
2 Gewichtsprozent Spindelöl
10 Gewichtsprozent Polyäthylenglycol
23 Teile Wasser

c) 20 Gewichtsprozent Wirkstoff
35 Gewichtsprozent Tonsil
8 Gewichtsprozent Zellpech
2 Gewichtsprozent Natriumsalz des N-Methyl-N-oleyl-taurins
35 Gewichtsprozent Kieselsäure

d) 20 Gewichtsprozent Wirkstoff
75 Gewichtsprozent Isophoron
5 Gewichtsprozent Kombinationsemulgator aus Calciumphenylsulfonat und Fettalkoholpolyglykoläther

Von den erfindungsgemäßen Verbindungen zeichnen sich durch eine überragende insektizide und ovizide Wirkung sowie durch große Pflanzenverträglichkeit insbesondere diejenigen aus, bei denen in der allgemeinen Formel I
$R_1$ Chlorphenyl, Dichlorphenyl, Bromphenyl, Fluorphenyl, Methoxyphenyl, Äthoxyphenyl, Trifluormethylphenyl, Methylphenyl, Naphthyl oder 1,3-Benzodioxol-5-yl,
$R_2$ Wasserstoff, Methyl, Fluor oder Chlor,
$R_3$ Wasserstoff, Methyl, Fluor oder Chlor,
$R_4$ Wasserstoff und $R_5$ Phenoxyphenyl, Fluor-phenoxyphenyl



Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berli
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Haman
Sitz der Gesellschaft: Berlin und Bergkamen · Handelsregister: AG Charlottenburg 93 HRB 283 und AG Kamen HRB 0061 · Berliner Commerzbank AG, Berlin, Konto-N
108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-N
2415008, Bankleitzahl 100 700 00 · Postscheckamt Berlin West, Konto-Nr. 1175-101, Bankleitzahl 100 100 10

oder Phenoxypyridyl darstellen.
Als herausragend wirksam ist 1-(4-Chlorphenyl)-1-(4-fluor-3-phenoxybenzyloxymethyl)-cyclopropan zu erwähnen.

Die erfindungsgemäßen Verbindungen lassen sich herstellen indem man eine Verbindung der allgemeinen Formel

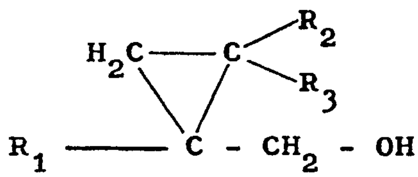

II

mit einer Verbindung der allgemeinen Formel

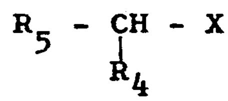

III

in Gegenwart einer Base umsetzt, worin $R_1$, $R_2$, $R_3$, $R_4$ und $R_5$ die oben genannte Bedeutung haben und X Halogen, Methansulfonat oder Toluolsulfonat darstellt.

Die Verätherung wird im allgemeinen in Lösung durchgeführt. Als Basen geeignet sind Metallalkoholate, wie zum Beispiel Kalium-tert.-butylat, Metallhydride, wie zum Beispiel Natriumhydrid, Metallamide, wie zum Beispiel Lithiumdiisopropylamid und Metallalkylverbindungen wie zum Beispiel Äthylmagnesiumbromid oder Butyllithium.

Als Lösungsmittel eignen sich gegenüber den Reaktanden, insbesondere den Basen, inerte Stoffe wie aliphatische und aromatische Kohlenwasserstoffe wie zum Beispiel Hexan, Benzol oder Toluol und Äther wie zum Beispiel Diäthyläther, Tetrahydrofuran oder Dimethoxyäthan; geeignet sind ferner Amide wie Dimethylformamid; bei Verwendung der Metallamide und Metallalkyle als Base kommen letztere aber zweckmäßigerweise erst nach der Deprotonierung der Alkoholkomponente als Kosolventien in Betracht.

-6-

Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen Handelsregister: AG Charlottenburg 93 HRB 283 und AG Kamen HRB 0061 · Berliner Commerzbank AG, Berlin, Konto-Nr. 108700600, Bankleitzahl 100 400 00 Berliner Handels- und Frankfurter Bank, Berlin Konto-Nr. 70045224, Bankleitzahl 100 202 00 Deutsche Bank Berlin AG, Konto-Nr. 2415008, Bankleitzahl 100 700 00 Postscheckamt Berlin West, Konto-Nr. 1175-101, Bankleitzahl 100 100 10

In einer anderen Ausführungsform wird die Verätherung in einem Zweiphasensystem unter Verwendung eines Katalysators und gegebenenfalls von Lösungsmitteln durchgeführt. Als Basen dienen Alkalihydroxide oder Alkalicarbonate, fest oder in wäßriger Lösung. Als Lösungsmittel geeignet sind die Reaktanden selbst, sofern sie flüssig sind; ansonsten werden mit Wasser nicht mischbare und gegenüber den Basen inerte Stoffe wie aliphatische oder aromatische Kohlenwasserstoffe wie zum Beispiel Hexan, Benzol oder Toluol verwendet. Als Katalysatoren kommen Verbindungen in Frage wie sie in Dehmlow u. Dehmlow, Phase Transfer Catalysis, Weinheim, 1980, beschrieben sind, vorzugsweise Kronenäther und quaternäre Ammoniumsalze.

Die Durchführung der Reaktionen erfolgt bei Temperaturen zwischen -78°C und 140°C, vorzugsweise bei 20-80°C, in der Regel bei Normaldruck.

Die Verbindungen mit $R_4$ = CN werden aus den entsprechenden Ausgangsverbindungen hergestellt, bei denen $R_4$ = H darstellt, welche mit N-Bromsuccinimid bromiert werden und man das Brom anschließend gegen Cyanid austauscht.

Die als Ausgangsstoffe zu verwendenden Alkohole lassen sich durch Reduktion der entsprechenden Carbonsäuren beziehungsweise Carbonsäureester darstellen.

Die Reduktion erfolgt nach an sich bekannten Methoden mit komplexen Metallhydriden wie Lithiumaluminiumhydrid oder Alkylaluminiumhydriden wie Diisobutylaluminiumhydrid. Die erforderlichen Carbonsäuren sind in der Literatur beschrieben oder lassen sich nach bekannten Methoden herstellen.
Die Benzylhalogenide beziehungsweise -tosylate und -mesylate sind ebenfalls in der Literatur beschrieben, beziehungsweise lassen sich nach bekannten Methoden herstellen.



Postanschrift: Schering Aktiengesellschaft, Postfach 65 03 11, D-1000 Berlin 65 · Fur Besucher: Berlin-Wedding, Müllerstraße 170-178 · Telegramme: Scheringchemie Berlin.
Vorstand Dr Herbert Asmis Dr Christian Bruhn, Dr. Heinz Hannse, Horst Kramp, Dr. Klaus Pohle, Dr. Horst Witzel · Vorsitzender des Aufsichtsrats: Hans-Jürgen Hamann Sitz der Gesellschaft Berlin und Bergkamen · Handelsregister: AG Charlottenburg 93 HRB 283 und AG Kamen HRB 0061 · Berliner Commerzbank AG, Berlin, Konto-Nr. 108700600 Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008 Bankleitzahl 100 700 00 · Postscheckamt Berlin West, Konto-Nr 1175-101, Bankleitzahl 100 100 10

Das folgende Beispiel erläutert die Herstellung der erfindungsgemäßen Verbindungen.

BEISPIEL 1

1-(4-Chlorphenyl)-1-(4-fluor-3-phenoxybenzyloxymethyl)-cyclopropan

0,44 g (10 mMol) Natriumhydrid-Dispersion (55%) werden durch dreimaliges Waschen mit trockenem Toluol vom Öl befreit. Es wird in 10 ml trockenem Dimethoxyäthan suspendiert und dann werden unter Rühren nacheinander 1,45 g (8,0mMol) 1-(4-Chlorphenyl)-1-hydroxymethyl-cyclopropan und 2,24 g (8,0mMol) 4-Fluor-3-phenoxybenzylbromid zugegeben. Die Temperatur steigt auf 33°C an. Nach fünfstündigem Rühren bei Raumtemperatur zeigt ein Dünnschichtchromatogramm, daß die Ausgangsmaterialien verschwunden sind und ein einheitliches Produkt entstanden ist.

Merck DC-Fertigplatten Kieselgel 60 $F_{254}$,
Laufmittel Hexan/Äther 8:2 $R_f$: 0,41

Man gießt jetzt auf Eiswasser, extrahiert zweimal mit Äther, wäscht die vereinigten Ätherphasen zweimal mit Wasser, trocknet mit Magnesiumsulfat und dampft ein. Es verbleiben 3,0 g eines farblosen Öls, das sind 98 % der Theorie.
Brechungsindex $n_D^{20}$: 1,5798

| | | | |
|---|---|---|---|
| Analyse: | Berechnet | C 72,15 % | H 5,27 % |
| | Gefunden | C 71,85 % | H 5,22 % |

In analoger Weise lassen sich die folgenden Verbindungen herstellen.

-8-

| Beispiel Nr. | Name der Verbindung | Physikalische Konstante |
|---|---|---|
| 2 | 1-(3-Phenoxybenzyloxymethyl)-1-phenylcyclopropan | $n_D^{20}$: 1,5836 |
| 3 | 1-(4-Methoxymethylbenzyloxymethyl)-1-phenylcyclopropan | $n_D^{20}$: 1,5522 |
| 4 | 1-(2,6-Dichlorbenzyloxymethyl)-1-phenylcyclopropan | $n_D^{20}$: 1,577 |
| 5 | 1-(4-Chlorphenyl)-1-(3-phenoxybenzyloxymethyl)-cyclopropan | $n_D^{20}$: 1,5874 |
| 6 | 1-(4-Chlorphenyl)-1-(4-methoxymethylbenzyloxymethyl)-cyclopropan | $n_D^{20}$: 1,5572 |
| 7 | 1-(4-Chlorphenyl)-1-(2,6-dichlorbenzyloxymethyl)-cyclopropan | $n_D^{20}$: 1,581 |
| 8 | 1-(3-Brom-4-fluorbenzyloxymethyl)-1-phenylcyclopropan | $n_D^{20}$: 1,5670 |
| 9 | 1-Phenyl-1-(3-trifluormethylbenzyloxymethyl)-cyclopropan | $n_D^{20}$: 1,5096 |
| 10 | 1-(4-Bromphenyl)-1-(3-phenoxybenzyloxymethyl)-cyclopropan | $n_D^{20}$: 1,5993 |
| 11 | 1-(1,3-Benzodioxol-5-yl)-1-(3-phenoxybenzyloxymethyl)-cyclopropan | $n_D^{20}$: 1,5870 |
| 12 | 1-(4-Äthoxyphenyl)-1-(3-phenoxybenzyloxymethyl)-cyclopropan | $n_D^{20}$: 1,5725 |
| 13 | 1-(1,3-Benzodioxol-5-yl)-1-(4-fluor-3-phenoxybenzyloxymethyl)-cyclopropan | $n_D^{20}$: 1,5769 |
| 14 | 1-(4-Äthoxyphenyl)-1-(4-fluor-3-phenoxybenzyloxymethyl)-cyclopropan | $n_D^{20}$: 1,5677 |
| 15 | 1-(4-Äthoxyphenyl)-1-(2-phenoxypyridin-6-ylmethoxymethyl)-cyclopropan | $n_D^{20}$: 1,5762 |
| 16 | 1-(3-Chlorphenyl)-1-(3-phenoxybenzyloxymethyl)-cyclopropan | $n_D^{20}$: 1,5890 |
| 17 | 1-(4-Methoxyphenyl)-1-(3-phenoxybenzyloxymethyl)-cyclopropan | $n_D^{20}$: 1,5792 |
| 18 | 1-(2,4-Dichlorphenyl)-1-(3-phenoxybenzyloxymethyl)-cyclopropan | $n_D^{20}$: 1,5905 |
| 19 | 1-(4-Methylphenyl)-1-(3-phenoxybenzyloxymethyl)-cyclopropan | $n_D^{20}$: 1,5768 |
| 20 | 1-(2-Methoxyphenyl)-1-(3-phenoxybenzyloxymethyl)-cyclopropan | $n_D^{20}$: 1,5790 |

-9-

| Beispiel Nr. | Name der Verbindung | Physikalische Konstante |
|---|---|---|
| 21 | 1-(4-Bromphenyl)-1-(4-fluor-3-phenoxybenzyloxymethyl)-cyclopropan | $n_D^{20}$: 1,5811 |
| 22 | 1-(4-Fluor-3-phenoxybenzyloxy-methyl)-1-(4-methoxyphenyl)-cyclopropan | $n_D^{20}$: 1,5748 |
| 23 | 2,2-Dichlor-1-(4-methoxyphenyl)-1-(3-phenoxybenzyloxymethyl)-cyclopropan | $n_D^{20}$: 1,5915 |
| 24 | 1-(4-Fluorphenyl)-1-(3-phenoxybenzyloxymethyl)-cyclopropan | $n_D^{20}$: 1,5692 |
| 25 | 1-(3,4-Dichlorphenyl)-1-(3-phenoxybenzyloxymethyl)-cyclopropan | $n_D^{20}$: 1,5937 |
| 26 | 1-(4-Fluorphenyl)-1-(4-fluor-3-phenoxybenzyloxymethyl)-cyclopropan | $n_D^{20}$: 1,5603 |
| 27 | 1-(3,4-Dichlorphenyl)-1-(4-fluor-3-phenoxybenzyloxy-methyl)-cyclopropan | $n_D^{20}$: 1,5838 |
| 28 | 1-(3,4-Dichlorphenyl)-1-(2-phenoxypyridin-6-ylmethoxy-methyl)-cyclopropan | $n_D^{20}$: 1,5952 |
| 29 | 1-(3-Phenoxybenzyloxymethyl)-1-(4-phenoxyphenyl)-cyclopropan | $n_D^{20}$: 1,6028 |
| 30 | 1-(4-Äthoxyphenyl)-2,2-dichlor-1-(3-phenoxybenzyloxy-methyl)-cyclopropan | $n_D^{20}$: 1,5849 |
| 31 | 1-(2,6-Difluorphenyl)-1-(3-phenoxybenzyloxymethyl)-cyclopropan | $n_D^{20}$: 1,5580 |
| 32 | 1-(2,6-Difluorphenyl)-1-(4-fluor-3-phenoxybenzyloxymethyl)-cyclopropan | $n_D^{20}$: 1,5500 |
| 33 | 1-(Naphth-1-yl)-1-(3-phenoxy-benzyloxymethyl)-cyclopropan | $n_D^{20}$: 1,6160 |

-10-

Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

| Beispiel Nr. | Name der Verbindung | Physikalische Konstante |
|---|---|---|
| 34 | 1-(Naphth-2-yl)-1-(3-phenoxy-benzyloxymethyl)-cyclopropan | $n_D^{20}$ : 1,16198 |
| 35 | 1-(4-Fluor-3-phenoxybenzyloxy-methyl)-1-(naphth-2-yl)-cyclopropan | $n_D^{20}$ : 1,6112 |
| 36 | 1-(3,4-Dimethoxyphenyl)-1-(3-phenoxybenzyloxymethyl)-cyclopropan | $n_D^{20}$ : 1,5853 |
| 37 | 1-(3,4-Dimethoxyphenyl)-1-(4-fluor-3-phenoxybenzyloxymethyl)-cyclopropan | $n_D^{20}$ : 1,5755 |
| 38 | 1-(4-Äthoxyphenyl)-2,2-di-chlor-1-(4-fluor-3-phenoxybenzyl-oxymethyl)-cyclopropan | $n_D^{20}$ : 1,5778 |
| 39 | 2,2-Dichlor-1-(4-fluor-3-phenoxybenzyloxymethyl)-1-(4-methoxyphenyl)-cyclopropan | $n_D^{20}$ : 1,5804 |
| 40 | 2,2-Dibrom-1-(4-fluor-3-phenoxybenzyloxymethyl)-1-(4-methoxyphenyl)-cyclopropan | $n_D^{20}$ : 1,6011 |
| 41 | 1-(4-Chlorphenyl)-1-(2-methyl-biphenyl-3-yl-methoxymethyl)-cyclopropan | $n_D^{20}$ : 1,5973 |

-11-

**Postanschrift: Schering Aktiengesellschaft, Postfach 65 03 11, D-1000 Berlin 65** · Für Besucher: Berlin-Wedding, Müllerstraße 170-178 · Telegramme: Scheringchemie Berlin.

Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

Die Verbindungen sind farb- und geruchlose Öle, die in praktisch allen organischen Lösungsmitteln gut löslich, in Wasser dagegen sehr schwer löslich sind.

Die folgenden Beispiele dienen zur Erläuterung der Anwendungsmöglichkeiten der erfindungsgemäßen Verbindungen, die in Form ihrer Zubereitungen erfolgte.

-12-

Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin

Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600 Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 11 75-101 Bankleitzahl 100 100 10

BEISPIEL 42

Fraßhemmende Wirkung auf Larven (L3) des Mexikanischen Bohnenkäfers (Epilachna varivestis)

Die erfindungsgemäßen Substanzen wurden als wäßrige Emulsionen mit der Wirkstoffkonzentration 0,1 % eingesetzt. In diese Wirkstoffzubereitungen wurden Buschbohnenpflanzen (Phaseolus vulgaris) im Primärblattstadium getaucht. Pro Versuchsglied wurden 2 Pflanzenstengel mit insgesamt 4 Primärblättern in mit Wasser gefüllte Glasvasen eingestellt und in Glaszylindern eingekäfigt. Danach wurden je 5 Larven des Mexikanischen Bohnenkäfers (Epilachna varivestis) im 3. Larvenstadium in die Glaszylinder eingezählt und für 3 Tage darin gehalten. Kriterium für die Wirkungsbeurteilung war die Fraßleistung in % der Fraßleistung der unbehandelten Kontrolle. Die erzielten Ergebnisse sind in nachstehender Tabelle zusammengefaßt.

-13-

| Erfindungsgemäße Verbindungen | Wirkstoffkonzentration in % | Fraßleistung in % |
|---|---|---|
| 1-(4-Chlorphenyl)-1-(4-fluor-3-phenoxybenzyloxymethyl)-cyclopropan | o,1 | 0 |
| 1-(4-Methoxymethylbenzyloxymethyl)-1-phenylcyclopropan | 0,1 | 50 |
| 1-(2,6-Dichlorbenzyloxymethyl)-1-phenylcyclopropan | 0,1 | 50 |
| 1-(4-Chlorphenyl)-1-(3-phenoxybenzyloxymethyl)-cyclopropan | 0,1 | 0 |
| 1-(4-Chlorphenyl)-1-(4-methoxymethylbenzyloxymethyl)-cyclopropan | 0,1 | 40 |
| 1-(4-Chlorphenyl)-1-(2,6-dichlorbenzyloxymethyl)-cyclopropan | 0,1 | 30 |
| 1-(3-Brom-4-fluorbenzyloxymethyl)-1-phenylcyclopropan | 0,1 | 30 |
| 1-Phenyl-1-(3-trifluormethylbenzyloxymethyl)-cyclopropan | 0,1 | 20 |
| 1-(4-Bromphenyl)-1-(3-phenoxybenzyloxymethyl)-cyclopropan | 0,1 | 0 |
| 1-(1,3-Benzodioxol-5-yl)-1-(3-phenoxybenzyloxymethyl)-cyclopropan | 0,1 | 0 |
| 1-(4-Äthoxyphenyl)-1-(3-phenoxybenzyloxymethyl)-cyclopropan | 0,1 | 0 |
| 1-(1,3-Benzodioxol-5-yl)-1-(4-fluor-3-phenoxybenzyloxymethyl)-cyclopropan | 0,1 | 0 |
| 1-(4-Äthoxyphenyl)-1-(4-fluor-3-phenoxybenzyloxymethyl)-cyclopropan | 0,1 | 0 |
| 1-(4-Äthoxyphenyl)-1-(2-phenoxypyridin-6-ylmethoxymethyl)-cyclopropan | 0,1 | 0 |
| 1-(3-Chlorphenyl)-1-(3-phenoxybenzyloxymethyl)-cyclopropan | 0,1 | 0 |
| 1-(4-Methoxyphenyl)-1-(3-phenoxybenzyloxymethyl)-cyclopropan | 0,1 | 0 |
| 1-(2,4-Dichlorphenyl)-1-(3-phenoxybenzyloxymethyl)-cyclopropan | 0,1 | 0 |
| 1-(4-Methylphenyl)-1-(3-phenoxybenzyloxymethyl)-cyclopropan | 0,1 | 0 |
| 1-(2-Methoxyphenyl)-1-(3-phenoxybenzyloxymethyl)-cyclopropan | 0,1 | 50 |

-14-

**Postanschrift: Schering Aktiengesellschaft, Postfach 65 03 11, D-1000 Berlin 65** · Für Besucher Berlin-Wedding, Müllerstraße 170-178 · Telegramme: Scheringchemie Berlin

Vorstand Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann Sitz der Gesellschaft Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr 70045224, Bankleitzahl 100 202 00 · Deutsche Bank Berlin AG, Konto-Nr. 2415008, Bankleitzahl 100 700 00 · Postscheckamt Berlin West, Konto-Nr 11 75-101, Bankleitzahl 100 100 10

| Erfindungsgemäße Verbindungen | Wirkstoffkonzentration in % | Fraßleistung in % |
|---|---|---|
| 1-(4-Bromphenyl)-1-(4-fluor-3-phenoxybenzyloxymethyl)-cyclopropan | 0,1 | 0 |
| 1-(4-Fluor-3-phenoxybenzyloxymethyl)-1-(4-methoxyphenyl)-cyclopropan | 0,1 | 0 |
| 2,2-Dichlor-1-(4-methoxyphenyl)-1-(3-phenoxybenzyloxymethyl)-cyclopropan | 0,1 | 0 |
| 1-(4-Fluorphenyl)-1-(3-phenoxybenzyloxymethyl)-cyclopropan | 0,1 | 0 |
| 1-(3,4-Dichlorphenyl)-1-(3-phenoxybenzyloxymethyl)-cyclopropan | 0,1 | 0 |
| 1-(4-Fluorphenyl)-1-(4-fluor-3-phenoxybenzyloxymethyl)-cyclopropan | 0,1 | 0 |
| 1-(3,4-Dichlorphenyl)-1-(4-fluor-3-phenoxybenzyloxymethyl)-cyclopropan | 0,1 | 0 |
| 1-(3,4-Dichlorphenyl)-1-(2-phenoxypyridin-6-ylmethyl)-cyclopropan | 0,1 | 30 |
| 1-(3-Phenoxybenzyloxymethyl)-1-(4-phenoxyphenyl)-cyclopropan | 0,1 | 20 |
| 1-(4-Äthoxyphenyl)-2,2-dichlor-1-(3-phenoxybenzyloxymethyl)-cyclopropan | 0,1 | 0 |
| 1-(2,6-Difluorphenyl)-1-(3-phenoxybenzyloxymethyl)-cyclopropan | 0,1 | 50 |
| 1-(2,6-Difluorphenyl)-1-(4-fluor-3-phenoxybenzyloxymethyl)-cyclopropan | 0,1 | 50 |
| 1-(Naphth-1-yl)-1-(3-phenoxybenzyloxymethyl)-cyclopropan | 0,1 | 50 |
| 1-(Naphth-2-yl)-1-(3-phenoxybenzyloxymethyl)-cyclopropan | 0,1 | 0 |
| 1-(4-Fluor-3-phenoxybenzyloxymethyl-1-(naphth-2-yl)-cyclopropan | 0,1 | 0 |
| 1-(3,4-Dimethoxyphenyl)-1-(3-phenoxybenzyloxymethyl)-cyclopropan | 0,1 | 20 |
| 1-(3,4-Dimethoxyphenyl)-1-(4-fluor-3-phenoxybenzyloxymethyl)-cyclopropan | 0,1 | 20 |
| 1-(4-Äthoxyphenyl)-2,2-dichlor-1-(4-fluor-3-phenoxybenzyloxymethyl)-cyclopropan | 0,1 | 0 |

-15-

Postanschrift: Schering Aktiengesellschaft, Postfach 65 03 11, D-1000 Berlin 65 · Fur Besucher: Berlin-Wedding, Müllerstraße 170-178 · Telegramme: Scheringchemie Berlin.

Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

| Erfindungsgemäße Verbindungen | Wirkstoffkonzentration in % | Fraßleistung in % |
|---|---|---|
| 2,2-Dichlor-1-(4-fluor-3-phenoxybenzyloxymethyl)-1-(4-methoxyphenyl)-cyclopropan | 0,1 | 0 |
| 2,2-Dibrom-1-(4-fluor-3-phenoxybenzyloxymethyl)-1-(4-methoxyphenyl)-cyclopropan | 0,1 | 0 |
| 1-(4-Chlorphenyl)-1-(2-methylbiphenyl-3-yl-methoxymethyl)-cyclopropan | 0,1 | 20 |
| Unbehandelte Kontrolle | - | 100 |

-16-

**BEISPIEL 43**

Ovizide Wirkung auf Eigelege der Ägyptischen Baumwolleule (Spodoptera littoralis)

Die erfindungsgemäßen Verbindungen sowie die Vergleichssubstanzen wurden als wäßrige Emulsionen mit den gewünschten Konzentrationen eingesetzt. In diese Wirkstoffzubereitungen wurden einen Tag alte Eigelege, die von befruchteten Falterweibchen auf Filterpapier abgesetzt worden waren, bis zur völligen Benetzung getaucht und für vier Tage in geschlossenen Petrischalen unter Langtagbedingungen deponiert. Kriterium für die Wirkungsbeurteilung war die prozentuale Schlupfverhinderung im Vergleich zu unbehandelten Eigelegen.
Die erzielten Ergebnisse sind in nachstehender Tabelle zusammengefaßt.

| Erfindungsgemäße Verbindungen | Wirkstoffkonzentration in % | Schlupfverhinderung in % |
|---|---|---|
| 1-(1,3-Benzodioxol-5-yl)-1-(4-fluor-3-phenoxybenzyloxymethyl)-cyclopropan | 0,016 | 90 |
| 1-(4-Äthoxyphenyl)-1-(4-fluor-3-phenoxybenzyloxymethyl)-cyclopropan | 0,016 | 100 |
| 1-(4-Chlorphenyl)-1-(4-fluor-3-phenoxybenzyloxymethyl)-cyclopropan | 0,016 | 90 |
| **Vergleichsmittel gemäß DE-OS 2709355** | | |
| 2-(4-Chlorphenyl)-3-methyl-1-(3-phenoxybenzyloxy)-butan | 0,016 | 27 |
| **Vergleichsmittel gemäß DE-OS 3117510** | | |
| 2-(4-Äthoxyphenyl)-2-methyl-1-(3-phenoxybenzyloxy)-propan | 0,016 | 40 |

-17-

Postanschrift: Schering Aktiengesellschaft, Postfach 65 03 11, D-1000 Berlin 65 · Fur Besucher: Berlin-Wedding, Mullerstraße 170-178 · Telegramme: Scheringchemie
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen H

BEISPIEL 44

Abtötende Wirkung auf Larven der Kohlschabe (Plutella xylostella)

Die erfindungsgemäße Verbindung sowie die Vergleichssubstanzen wurden als wäßrige Emulsionen mit der Wirkstoffkonzentration 0,001 % eingesetzt. Mit diesen Wirkstoffzubereitungen wurde je ein Blumenkohlblättchen pro Versuchsglied mit 4 mg Spritzbrühe/$cm^2$ in Polystrol-Petrischalen dosiert gespritzt. Nach dem Antrocknen der Spritzbeläge wurden in jede Petrischale 5 Jungraupen der Kohlschabe (Plutella xylostella) eingezählt und für 2 Tage dem behandelten Futter im Labor unter Langtagbedingungen exponiert. Kriterium für die Wirkungsbeurteilung war die Mortalität der Raupen nach 2 Tagen.

Die erzielten Ergebnisse sind in nachstehender Tabelle zusammengefaßt.

| Erfindungsgemäße Verbindung | Wirkstoffkonzentration in % | Wirkung in % |
|---|---|---|
| 1-(4-Chlorphenyl)-1-(4-fluor-3-phenoxybenzyloxymethyl)-cyclopropan | 0,001 | 100 |
| Vergleichsmittel gemäß DE-OS 2709355 | | |
| 2-(4-Chlorphenyl)-3-methyl-1-(3-phenoxybenzyloxy)-propan | 0,001 | 0 |
| Vergleichsmittel gemäß DE-OS 3117510 | | |
| 2-(4-Äthoxyphenyl)-2-methyl-1-(3-phenoxybenzyloxy)-propan | 0,001 | 50 |

-18-

BEISPIEL 45

Abtötende Wirkung auf Adulte des Kornkäfers (Sitophilus granarius)

Die erfindungsgemäßen Verbindungen sowie die Vergleichssubstanzen wurden als wäßrige Emulsionen mit der Wirkstoffkonzentration 0,1 % eingesetzt. Mit diesen Wirkstoffzubereitungen wurden die Böden von Polystyrol-Petrischalen dosiert (4 mg Spritzbrühe/$cm^2$) gespritzt. Nach Trocknung der Spritzbeläge wurden für vier Tage je 100 adulte Kornkäfer (Sitophilus granarius) diesen Spritzbelägen in geschlossenen Petrischalen im Dunkelraum exponiert. Kriterium für die Wirksamkeit war die Mortalität der Käfer nach vier Tagen.

Die nachfolgende Tabelle stellt die Ergebnisse vergleichend dar.

| Erfindungsgemäße Verbindungen | Wirkstoffkonzentration in % | Wirkung in % |
|---|---|---|
| 1-(1,3-Benzodioxol-5-yl)-1-(4-fluor-3-phenoxybenzyloxymethyl)-cyclopropan | 0,1 | 100 |
| 1-(4-Äthoxyphenyl)-1-(4-fluor-3-phenoxybenzyloxymethyl)-cyclopropan | 0,1 | 100 |
| 1-(4-Chlorphenyl)-1-(4-fluor-3-phenoxybenzyloxymethyl)-cyclopropan | 0,1 | 100 |
| Vergleichsmittel gemäß DE-OS 2709355 | | |
| 2-(4-Chlorphenyl)-3-methyl-1-(3-phenoxybenzyloxy)-butan | 0,1 | 0 |
| Vergleichsmittel gemäß DE-OS 3117510 | | |
| 2-(4-Äthoxyphenyl)-2-methyl-1-(3-phenoxybenzyloxy)-propan | 0,1 | 67 |

-19-

BEISPIEL 46

Abtötende Wirkung auf Larven (L2) der Ägyptischen Baumwolleule (Spodoptera Littoralis)

Die erfindungsgemäßen Verbindungen sowie die Vergleichssubstanzen wurden als wäßrige Emulsionen mit der Wirkstoffkonzentration 0,001 % eingesetzt. Mit diesen Wirkstoffzubereitungen wurden je ein Fiederblattpaar der Puffbohne (Vicia faba) sowie 10 Larven (L2) der Ägyptischen Baumwolleule (Spodoptera littoralis) pro Versuchsglied mit 4 mg Spritzbrühe/$cm^2$ in Polystyrol-Petrischalen dosiert gespritzt. Die geschlossenen Petrischalen wurden dann im Labor unter Langtagbedingungen für zwei Tage aufgestellt. Kriterium für die Wirkungsbeurteilung war die Mortalität der Larven nach zwei Tagen.

Die nachstehende Tabelle faßt die erzielten Ergebnisse zusammen.

| Erfindungsgemäße Verbindungen | Wirkstoffkonzentration in % | Wirkung in % |
|---|---|---|
| 1-(4-Äthoxyphenyl)-1-(4-fluor-3-phenoxybenzyloxymethyl)-cyclopropan | 0,001 | 80 |
| 1-(4-Chlorphenyl)-1-(4-fluor-3-phenoxybenzyloxymethyl)-cyclopropan | 0,001 | 90 |
| **Vergleichsmittel gemäß DE-OS 2709355** | | |
| 2-(4-Chlorphenyl)-3-methyl-1-(3-phenoxybenzyloxy)-butan | 0,001 | 0 |
| **Vergleichsmittel gemäß DE-OS 3117510** | | |
| 2-(4-Äthoxyphenyl)-2-methyl-1-(3-phenoxybenzyloxy)-propan | 0,001 | 50 |

-20-

# PATENTANSPRÜCHE

1. Arylmethylätherderivate der allgemeinen Formel

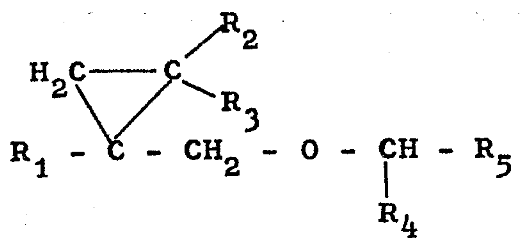

I,

in der

$R_1$ Aryl, durch $C_1$-$C_4$-Alkyl, Halogen-$C_1$-$C_4$-alkyl, Phenyl-$C_1$-$C_4$-alkyl, $C_2$-$C_4$-Alkenyl, Halogen-$C_2$-$C_4$-alkenyl, Phenyl-$C_2$-$C_4$-alkenyl, $C_2$-$C_4$-Alkinyl, Halogen-$C_2$-$C_4$-alkinyl, Phenyl-$C_2$-$C_4$-alkinyl,$C_1$-$C_4$-Alkoxy, Halogen-$C_1$-$C_4$-alkoxy, Phenyl-$C_1$-$C_4$-alkoxy, $C_2$-$C_4$-Alkenyloxy, Halogen-$C_2$-$C_4$-alkenyloxy, Phenyl-$C_2$-$C_4$-alkenyloxy, Halogen, Cyan, Nitro, Aryloxy, Halogenaryloxy, $C_1$-$C_4$-Alkyl-aryloxy oder Nitro-$C_1$-$C_4$-alkyl-aryloxy substituiertes Aryl,

$R_2$ Wasserstoff, Methyl oder Halogen,

$R_3$ Wasserstoff, Methyl oder Halogen,

$R_4$ Wasserstoff, Cyano oder Äthinyl,

$R_5$ Phenyl, Pyridyl oder durch $C_1$-$C_6$-Alkyl, Halogen-$C_1$-$C_6$-alkyl, Phenyl-$C_1$-$C_6$-alkyl, durch O-, N- oder S-Atome unterbrochenes $C_1$-$C_6$-Alkyl, $C_2$-$C_4$-Alkenyl, Halogen-$C_2$-$C_4$-alkenyl, Phenyl-$C_2$-$C_4$-alkenyl, $C_1$-$C_4$-Alkoxy, Halogen-$C_1$-$C_4$-alkoxy, Phenyl-$C_1$-$C_4$-alkoxy, $C_2$-$C_4$-Alkenyloxy, Halogen-$C_2$-$C_4$-alkenyloxy, Phenyl-$C_2$-$C_4$-alkenyloxy, Aryloxy, Halogenaryloxy, $C_1$-$C_4$-Alkyl-aryloxy, Arylamino, Halogenarylamino, $C_1$-$C_4$-Alkylarylamino, Aryl-N-$C_1$-$C_4$-alkyl-amino, Aryl-N-$C_1$-$C_4$-acyl-amino, Aroyl, Halogenaroyl, $C_1$-$C_4$-Alkylaroyl, Aryl, Halogenaryl, $C_1$-$C_4$-Alkylaryl oder Halogen ein- oder mehrfach substituiertes Phenyl oder Pyridyl darstellen.

-21-

**Postanschrift: Schering Aktiengesellschaft, Postfach 65 03 11, D-1000 Berlin 65** · Für Besucher: Berlin-Wedding, Müllerstraße 170-178 · Telegramme: Scheringchemie Berlin.

Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 1175-101, Bankleitzahl 100 100 10

2. Arylmethylätherderivate gemäß Anspruch 1, worin
$R_1$ Chlorphenyl, Dichlorphenyl, Bromphenyl, Fluorphenyl, Methoxyphenyl, Äthoxyphenyl, Trifluormethylphenyl, Methylphenyl, Naphthyl oder 1,3-Benzodioxol-5-yl,
$R_2$ Wasserstoff, Methyl, Fluor oder Chlor,
$R_3$ Wasserstoff, Methyl, Fluor oder Chlor,
$R_4$ Wasserstoff und
$R_5$ Phenoxyphenyl, Fluor-phenoxyphenyl oder Phenoxy-pyridyl darstellen.

3. 1-(4-Chlorphenyl)-1-(4-fluor-3-phenoxybenzyloxymethyl)-cyclopropan

4. 1-(1,3-Benzodioxol)-1-(3-phenoxybenzyloxymethyl)-cyclopropan

5. 1-(4-Äthoxyphenyl)-1-(4-fluor-3-phenoxybenzyloxymethyl)-cyclopropan

6. Verfahren zur Herstellung von Arylmethylätherderivaten gemäß Ansprüchen 1 bis 5, dadurch gekennzeichnet, daß man eine Verbindung der allgemeinen Formel

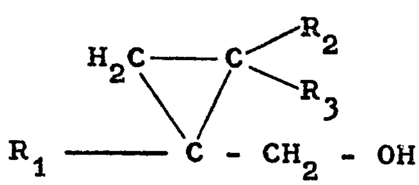

II

mit einer Verbindung der allgemeinen Formel

$$R_5 - \underset{\displaystyle R_4}{\underset{|}{CH}} - X$$

III

in Gegenwart einer Base umsetzt, worin $R_1$, $R_2$, $R_3$, $R_4$ und $R_5$ die oben genannte Bedeutung haben und X Halogen, Methansulfonat oder Toluolsulfonat darstellt.

-22-

Postanschrift: Schering Aktiengesellschaft, Postfach 65 03 11, D-1000 Berlin 65 · Für Besucher: Berlin-Wedding, Müllerstraße 170-178 · Telegramme: Scheringchemie Berlin
Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 11 75-101, Bankleitzahl 100 100 10

7. Schädlingsbekämpfungsmittel, gekennzeichnet durch einen Gehalt an mindestens einer Verbindung gemäß Ansprüchen 1 bis 5.

8. Mittel zur Bekämpfung von Insekten gemäß Anspruch 7.

9. Schädlingsbekämpfungsmittel gemäß Anspruch 7 in Mischung mit Träger und/oder Hilfsstoffen.

10. Schädlingsbekämpfungsmittel gemäß Anspruch 7 hergestellt nach Verfahren gemäß Anspruch 6.



**Postanschrift: Schering Aktiengesellschaft, Postfach 65 03 11, D-1000 Berlin 65** · Für Besucher: Berlin-Wedding, Müllerstraße 170-178 · Telegramme: Scheringchemie Berlin

Vorstand: Dr. Herbert Asmis, Dr. Christian Bruhn, Dr. Heinz Hannse, Horst Kramp, Dr. Klaus Pohle, Dr. Horst Witzel · Vorsitzender des Aufsichtsrats: Hans-Jürgen Hamann
Sitz der Gesellschaft: Berlin und Bergkamen · Handelsregister: AG Charlottenburg 93 HRB 283 und AG Kamen HRB 0061 · Berliner Commerzbank AG, Berlin, Konto-Nr. 108700600, Bankleitzahl 100 400 00 · Berliner Handels- und Frankfurter Bank, Berlin, Konto-Nr. 70045224, Bankleitzahl 100 202 00 · Deutsche Bank Berlin AG, Konto-Nr. 2415008, Bankleitzahl 100 700 00 · Postscheckamt Berlin West, Konto-Nr. 11 75-101, Bankleitzahl 100 100 10

Formular Nr. 1439 4